(19) Europäisches Patentamt
European Patent Office
Office européen des brevets

(11) EP 2 166 020 A1

(12) EUROPEAN PATENT APPLICATION
published in accordance with Art. 153(4) EPC

(43) Date of publication:
**24.03.2010 Bulletin 2010/12**

(21) Application number: **08790697.0**

(22) Date of filing: **27.06.2008**

(51) Int Cl.:
***C07K 14/47*** *(2006.01)* ***A61K 38/00*** *(2006.01)*
***A61P 9/12*** *(2006.01)* ***A61P 43/00*** *(2006.01)*
***C07K 7/06*** *(2006.01)* ***C07K 7/08*** *(2006.01)*

(86) International application number:
**PCT/JP2008/061732**

(87) International publication number:
**WO 2009/001928 (31.12.2008 Gazette 2009/01)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **28.06.2007 JP 2007170550**

(71) Applicant: **Asubio Pharma Co., Ltd.**
**Minato-ku**
**Tokyo 107-8541 (JP)**

(72) Inventors:
- **OKAMOTO, Atsushi**
  **Mishima-gun**
  **Osaka 618-8503 (JP)**
- **MAGOTA, Koji**
  **Ohra-gun**
  **Gunma 370-0503 (JP)**
- **HAYASHI, Yujiro**
  **Ohra-gun**
  **Gunma 370-0503 (JP)**

(74) Representative: **Vossius, Volker**
**Dr. Volker Vossius**
**Patent- und Rechtsanwaltskanzlei**
**Geibelstrasse 6**
**81679 München (DE)**

(54) **PEPTIDE HAVING HYPOTENSIVE ACTIVITY**

(57) The present invention provides a novel peptide having a physiological activity. Because of having a hypotensive activity, this novel peptide is useful in treating a disease caused by hypertension. Also, an antibody to the novel peptide is provided.

EP 2 166 020 A1

SEQUENCE LISTING

```
<110>  Asubio Pharma Co., Ltd.

<120>  Peptide Having Hypotensive Activity

<130>  R3106 EP

<150>  2007-170550
<151>  2007-6-28

<160>  43

<170>  PatentIn version 3.1

<210>  1
<211>  10
<212>  PRT
<213>  Artificial sequence

<220>
<223>  /note="Description of artificial sequence: Compound 24"

<400>  1

Leu Phe Phe Arg Arg Leu Gln Ala Tyr Phe
1               5                   10


<210>  2
<211>  21
<212>  PRT
<213>  Artificial sequence

<220>
<223>  /note="Description of artificial sequence: synthetic peptide sequence"

<400>  2

Asn Gly Ala Thr Val Arg Asn Glu Asp Lys Trp Lys Pro Leu Asn Asn
1               5                   10                  15


Pro Arg Asn Arg Asp
            20


<210>  3
<211>  100
<212>  PRT
<213>  Homo sapiens

<400>  3

Met Thr Arg Ala Pro Leu Leu Leu Leu Cys Val Ala Leu Val Leu Leu
1               5                   10                  15


Gly His Val Asn Gly Ala Thr Val Arg Asn Glu Asp Lys Trp Lys Pro
            20                  25                  30


Leu Asn Asn Pro Arg Asn Arg Asp Leu Phe Phe Arg Arg Leu Gln Ala
        35                  40                  45


Tyr Phe Lys Gly Arg Gly Leu Asp Leu Gly Thr Phe Pro Asn Pro Phe
```

50 55 60

Pro Thr Asn Glu Asn Pro Arg Pro Leu Ser Phe Gln Ser Glu Leu Thr
65 70 75 80

Ala Ser Ala Ser Ala Asp Tyr Glu Glu Gln Lys Asn Ser Phe His Asn
85 90 95

Tyr Leu Lys Gly
100

<210> 4
<211> 300
<212> DNA
<213> Homo sapiens

<400> 4
atgaccagag cacctctcct gctactatgt gttgccctgg tgctgcttgg gcatgtgaat 60

ggagccacag taagaaatga ggacaaatgg aagccactca acaaccccag aaacagagat 120

ctgtttttca gaaggcttca ggcatatttt aagggcagag gtcttgatct tggaacattt 180

ccaaatcctt tccccacgaa tgaaaatcct agacctctct ctttccagtc agaacttact 240

gcttctgcat ctgcagatta tgaagagcag aaaaactcct ttcacaatta tctcaaaggc 300

<210> 5
<211> 47
<212> PRT
<213> Homo sapiens

<400> 5

Gly Leu Asp Leu Gly Thr Phe Pro Asn Pro Phe Pro Thr Asn Glu Asn
1 5 10 15

Pro Arg Pro Leu Ser Phe Gln Ser Glu Leu Thr Ala Ser Ala Ser Ala
20 25 30

Asp Tyr Glu Glu Gln Lys Asn Ser Phe His Asn Tyr Leu Lys Gly
35 40 45

<210> 6
<211> 22
<212> PRT
<213> Artificial sequence

<220>
<223> /note="Description of artificial sequence: Compound 1 (HC021-001)"

<400> 6

Ala Thr Val Arg Asn Glu Asp Lys Trp Lys Pro Leu Asn Asn Pro Arg
1 5 10 15

Asn Arg Asp Leu Phe Phe
20

```
<210>  7
<211>  25
<212>  PRT
<213>  Artificial sequence

<220>
<223>  /note="Description of artificial sequence: Compound 2 (HC021-002)"

<400>  7

Leu Gln Ala Tyr Phe Lys Gly Arg Gly Leu Asp Leu Gly Thr Phe Pro
1               5                   10                  15

Asn Pro Phe Pro Thr Asn Glu Asn Pro
            20                  25


<210>  8
<211>  14
<212>  PRT
<213>  Artificial sequence

<220>
<223>  /note="Description of artificial sequence: Compound 3 (HC021-004),
Compound 15, Compound 32"

<400>  8

Asn Arg Asp Leu Phe Phe Arg Arg Leu Gln Ala Tyr Phe Lys
1               5                   10


<210>  9
<211>  6
<212>  PRT
<213>  Artificial sequence

<220>
<223>  /note="Description of artificial sequence: Compound 4 (HC021-006)"

<400>  9

Leu Gln Ala Tyr Phe Lys
1               5


<210>  10
<211>  30
<212>  PRT
<213>  Artificial sequence

<220>
<223>  /note="Description of artificial sequence: Compound 5 (HC021-007),
Compound 8, Compound 31"

<400>  10

Ala Thr Val Arg Asn Glu Asp Lys Trp Lys Pro Leu Asn Asn Pro Arg
1               5                   10                  15

Asn Arg Asp Leu Phe Phe Arg Arg Leu Gln Ala Tyr Phe Lys
            20                  25                  30


<210>  11
```

```
<211>  13
<212>  PRT
<213>  Artificial sequence

<220>
<223>  /note="Description of artificial sequence: Compound 16"

<400>  11

Arg Asp Leu Phe Phe Arg Arg Leu Gln Ala Tyr Phe Lys
1               5                   10


<210>  12
<211>  12
<212>  PRT
<213>  Artificial sequence

<220>
<223>  /note="Description of artificial sequence: Compound 17"

<400>  12

Asp Leu Phe Phe Arg Arg Leu Gln Ala Tyr Phe Lys
1               5                   10


<210>  13
<211>  11
<212>  PRT
<213>  Artificial sequence

<220>
<223>  /note="Description of artificial sequence: Compound 18/19"

<400>  13

Leu Phe Phe Arg Arg Leu Gln Ala Tyr Phe Lys
1               5                   10


<210>  14
<211>  10
<212>  PRT
<213>  Artificial sequence

<220>
<223>  /note="Description of artificial sequence: Compound 20"

<400>  14

Phe Phe Arg Arg Leu Gln Ala Tyr Phe Lys
1               5                   10


<210>  15
<211>  9
<212>  PRT
<213>  Artificial sequence

<220>
<223>  /note="Description of artificial sequence: Compound 21"

<400>  15

Phe Arg Arg Leu Gln Ala Tyr Phe Lys
1               5
```

```
<210>  16
<211>  8
<212>  PRT
<213>  Artificial sequence

<220>
<223>  /note="Description of artificial sequence: Compound 22"

<400>  16

Arg Arg Leu Gln Ala Tyr Phe Lys
1               5


<210>  17
<211>  7
<212>  PRT
<213>  Artificial sequence

<220>
<223>  /note="Description of artificial sequence: Compound 23"

<400>  17

Arg Leu Gln Ala Tyr Phe Lys
1               5


<210>  18
<211>  29
<212>  PRT
<213>  Artificial sequence

<220>
<223>  /note="Description of artificial sequence: Compound 9/10"

<400>  18

Ala Thr Val Arg Asn Glu Asp Lys Trp Lys Pro Leu Asn Asn Pro Arg
1               5                   10                  15


Asn Arg Asp Leu Phe Phe Arg Arg Leu Gln Ala Tyr Phe
            20                  25


<210>  19
<211>  31
<212>  PRT
<213>  Artificial sequence

<220>
<223>  /note="Description of artificial sequence: Compound 7"

<400>  19

Gly Ala Thr Val Arg Asn Glu Asp Lys Trp Lys Pro Leu Asn Asn Pro
1               5                   10                  15


Arg Asn Arg Asp Leu Phe Phe Arg Arg Leu Gln Ala Tyr Phe Lys
            20                  25                  30


<210>  20
<211>  32
<212>  PRT
```

<213> Artificial sequence

<220>
<223> /note="Description of artificial sequence: Compound 6"

<400> 20

```
Asn Gly Ala Thr Val Arg Asn Glu Asp Lys Trp Lys Pro Leu Asn Asn
1               5                   10                  15

Pro Arg Asn Arg Asp Leu Phe Phe Arg Arg Leu Gln Ala Tyr Phe Lys
            20                  25                  30
```

<210> 21
<211> 15
<212> PRT
<213> Artificial sequence

<220>
<223> /note="Description of artificial sequence: Compound 11/12"

<400> 21

```
Asn Arg Asp Leu Phe Phe Arg Arg Leu Gln Ala Tyr Phe Lys Gly
1               5                   10                  15
```

<210> 22
<211> 16
<212> PRT
<213> Artificial sequence

<220>
<223> /note="Description of artificial sequence: Compound 13/14"

<400> 22

```
Asn Arg Asp Leu Phe Phe Arg Arg Leu Gln Ala Tyr Phe Lys Gly Arg
1               5                   10                  15
```

<210> 23
<211> 9
<212> PRT
<213> Artificial sequence

<220>
<223> /note="Description of artificial sequence: Compound 25, active center"

<400> 23

```
Leu Phe Phe Arg Arg Leu Gln Ala Tyr
1               5
```

<210> 24
<211> 8
<212> PRT
<213> Artificial sequence

<220>
<223> /note="Description of artificial sequence: Compound 26"

<400> 24

```
Leu Phe Phe Arg Arg Leu Gln Ala
```

1 5

<210> 25
<211> 24
<212> PRT
<213> Artificial sequence

<220>
<223> /note="Description of artificial sequence: Compound 27/28"

<400> 25

Leu Phe Phe Arg Arg Leu Gln Ala Tyr Phe Lys Gly Arg Gly Leu Asp
1 5 10 15

Leu Gly Thr Phe Pro Asn Pro Phe
20

<210> 26
<211> 31
<212> PRT
<213> Artificial sequence

<220>
<223> /note="Description of artificial sequence: Compound 29/30"

<400> 26

Leu Phe Phe Arg Arg Leu Gln Ala Tyr Phe Lys Gly Arg Gly Leu Asp
1 5 10 15

Leu Gly Thr Phe Pro Asn Pro Phe Pro Thr Asn Glu Asn Pro Arg
20 25 30

<210> 27
<211> 25
<212> PRT
<213> Artificial sequence

<220>
<223> /note="Description of artificial sequence: Compound 33"

<400> 27

Glu Asp Lys Trp Lys Pro Leu Asn Asn Pro Arg Asn Arg Asp Leu Phe
1 5 10 15

Phe Arg Arg Leu Gln Ala Tyr Phe Lys
20 25

<210> 28
<211> 30
<212> PRT
<213> Artificial sequence

<220>
<223> /note="Description of artificial sequence: Compound 34"

<400> 28

Ala Thr Leu Arg Asn Glu Asp Thr Trp Lys Pro Leu Ser Asn Pro Arg

1 5 10 15

Asn Arg Asp Leu Phe Phe Arg Arg Leu Gln Ala Tyr Phe Lys
20 25 30

<210> 29
<211> 30
<212> PRT
<213> Artificial sequence

<220>
<223> /note="Description of artificial sequence: Compound 35"

<400> 29

Ala Thr Leu Gly Ser Glu Asp Thr Trp Lys Pro Leu Ser Asn Pro Arg
1 5 10 15

Asn Arg Asp Leu Phe Phe Arg Arg Leu Gln Ala Tyr Phe Lys
20 25 30

<210> 30
<211> 30
<212> PRT
<213> Artificial sequence

<220>
<223> /note="Description of artificial sequence: Compound 36"

<400> 30

Ala Thr Leu Arg Asn Glu Asp Thr Trp Lys Pro Leu Ser Asn Pro Arg
1 5 10 15

Asn Arg Glu Leu Phe Phe Arg Ser Leu Gln Ala Tyr Phe Lys
20 25 30

<210> 31
<211> 30
<212> PRT
<213> Artificial sequence

<220>
<223> /note="Description of artificial sequence: Compound 37"

<400> 31

Ala Thr Leu Gly Ser Glu Asp Thr Trp Lys Pro Leu Ser Asn Pro Arg
1 5 10 15

Asn Arg Glu Leu Phe Phe Arg Ser Leu Gln Ala Tyr Phe Lys
20 25 30

<210> 32
<211> 29
<212> PRT
<213> Artificial sequence

<220>
<223> /note="Description of artificial sequence: Compound 38"

<400> 32

Ala Thr Leu Gly Ser Glu Asp Thr Trp Lys Pro Leu Ser Asn Pro Arg
1 5 10 15

Asn Arg Glu Leu Phe Phe Arg Ser Leu Gln Ala Tyr Phe
20 25

<210> 33
<211> 28
<212> PRT
<213> Artificial sequence

<220>
<223> /note="Description of artificial sequence: Compound 39"

<400> 33

Phe Val Pro Ile Phe Thr Tyr Gly Glu Leu Gln Arg Met Gln Asn Arg
1 5 10 15

Asp Leu Phe Phe Arg Arg Leu Gln Ala Tyr Phe Lys
20 25

<210> 34
<211> 31
<212> PRT
<213> Artificial sequence

<220>
<223> /note="Description of artificial sequence: Compound 40"

<400> 34

Val Gln Gln Arg Lys Glu Ser Lys Lys Pro Pro Ala Lys Leu Gln Pro
1 5 10 15

Arg Asn Arg Asp Leu Phe Phe Arg Arg Leu Gln Ala Tyr Phe Lys
20 25 30

<210> 35
<211> 14
<212> PRT
<213> Artificial sequence

<220>
<223> /note="Description of artificial sequence: Compound 41"

<400> 35

Asn Arg Glu Leu Phe Phe Arg Arg Leu Gln Ala Tyr Phe Lys
1 5 10

<210> 36
<211> 14
<212> PRT
<213> Artificial sequence

<220>
<223> /note="Description of artificial sequence: Compound 42"

<400> 36

Asn Arg Asp Leu Phe Phe Arg Arg Leu Gln Ala Tyr Ala Lys
1 5 10

<210> 37
<211> 11
<212> PRT
<213> Artificial sequence

<220>
<223> /note="Description of artificial sequence: Compound 43"

<400> 37

Leu Phe Phe Arg Ser Leu Gln Ala Tyr Phe Lys
1 5 10

<210> 38
<211> 11
<212> PRT
<213> Artificial sequence

<220>
<223> /note="Description of artificial sequence: Compound 44"

<400> 38

Leu Phe Phe Arg Arg Leu Gln Ala Leu Phe Lys
1 5 10

<210> 39
<211> 11
<212> PRT
<213> Artificial sequence

<220>
<223> /note="Description of artificial sequence: Compound 45"

<220>
<221> MISC_FEATURE
<222> (9)..(9)
<223> Xaa: cyclohexylalanine

<400> 39

Leu Phe Phe Arg Arg Leu Gln Ala Xaa Phe Lys
1 5 10

<210> 40
<211> 11
<212> PRT
<213> Artificial sequence

<220>
<223> /note="Description of artificial sequence: Compound 46"

<400> 40

Leu Phe Phe Arg Arg Leu Gln Ala Trp Phe Lys
1 5 10

```
<210>  41
<211>  11
<212>  PRT
<213>  Artificial sequence

<220>
<223>  /note="Description of artificial sequence: Compound 47"

<400>  41

Leu Phe Phe Arg Arg Leu Gln Ala His Phe Lys
1               5                   10


<210>  42
<211>  26
<212>  PRT
<213>  Artificial sequence

<220>
<223>  /note="Description of artificial sequence: Compound 48"

<400>  42

Leu Phe Phe Arg Arg Leu Gln Ala Tyr Phe Lys Asn Gly Ala Glu Asp
1               5                   10                  15

Glu Ser Ala Glu Ala Phe Pro Leu Glu Phe
            20                  25


<210>  43
<211>  30
<212>  PRT
<213>  Artificial sequence

<220>
<223>  /note="Description of artificial sequence: Compound 49"

<400>  43

Leu Phe Phe Arg Arg Leu Gln Ala Tyr Phe Lys Ala Ser Ala Ser Ala
1               5                   10                  15

Asp Tyr Glu Glu Gln Lys Asn Ser Phe His Asn Tyr Leu Lys
            20                  25                  30
```

## Description

TECHNICAL FIELD

The present invention relates to a novel endogenous peptide having a hypotensive activity or an analog thereof.

BACKGROUND ART

Studies regarding an endogenous physiologically active peptide have started with identification of insulin in 1921. To date, a large number of endogenous peptides have been identified in higher organisms including humans (Non-Patent Document 1). These endogenous physiologically active peptides do not have a single physiological action, but they may exhibit different actions depending on the type of a target organ in many cases. For example, ghrelin has been known to have various functions, such that it acts on the hypophysis and exhibits an action to promote the secretion of growth hormone that is another type of endogenous physiologically active peptide, and such that it acts on the hypothalamus and exhibits a food intake promoting action (Non-Patent Document 2). In addition, adrenomedullin has been known to have various functions, such that it acts on the blood vessel and exhibits a vasodilator action, and such that it acts on the hypophysis to suppress the secretion of ACTH that is another type of endogenous physiologically active peptide (Non-Patent Document 3).

Moreover, studies regarding endogenous physiologically active peptides other than the aforementioned ghrelin and adrenomedullin have also been progressing. The facts that a certain physiologically active peptide controls the expression of the action of another peptide and that multiple peptides mutually control their actions to form a complicated network so as to control the mechanisms of living bodies have been clarified. For instance, it has been reported that, for food intake and energy balance in living bodies, endogenous physiologically active peptides having a food intake promoting action, such as ghrelin, Agouti-related peptide and neuropeptide Y, and peptides having a food intake suppressing action, such as peptide YY, cholecystokinin, leptin, melanocortin and insulin transmit information between the peripheral nerve and the central nerve, so as to mutually control their actions (Non-Patent Document 4).

On the other hand, such endogenous physiologically active peptides are used in the treatment of disease. For example, insulin is used as an antidiabetes agent, an active fragment of parathyroid hormone is used as a therapeutic agent for osteoporosis, and atrial natriuretic peptide (ANP) is used as a therapeutic agent for acute heart failure. Pharmaceutical agents using these endogenous physiologically active peptides have an action mechanism, which is hardly replaced with low molecular weight compounds, and utilize substances originally existing in living bodies to normalize the mechanisms of the living bodies. Thus, such pharmaceutical agents are considered to be excellent in terms of safety.

Accordingly, identification of a novel endogenous physiologically active peptide leads not only to the discovery of a novel substance having a physiological activity, but also to clarification of a novel mechanism of living body. Further, it would lead to the development of a novel method for treating disease based on a novel action mechanism.

As a common method for identifying an endogenous physiologically active peptide, a method for fractionating and/or purifying a peptide from a living tissue extract using an action on various evaluation systems as an indicator has been known. To date, as such evaluation systems, an action on an excised organ, such as the relaxation and contraction of the smooth muscle of the intestinal canal or the like (Non-Patent Documents 5 and 6); the fluctuation of intracellular second messengers such as cAMP and Ca ions in cultured cells (Non-Patent Document 7); and the like have been utilized. Utilizing such evaluation systems, novel physiologically active peptides have been identified. In recent years, a receptor having an unknown ligand that had been identified or predicted by genomic analysis has been forced to express in the cultured cells, and the fluctuation of intracellular second messengers (Non-Patent Documents 8 and 9), a change in the amount of an extracellular arachidonic acid metabolite (Non-Patent Document 10), a change in an extracellular acidification rate (Non-Patent Document 11), etc. have been utilized as evaluation systems, so as to identify novel endogenous physiologically active peptides. Moreover, there have been utilized evaluation systems using most-advanced science and technology, such as a change in the intracellular localization of a receptor or a receptor-binding protein (Non-Patent Document 12) and a change in an intracellular dielectric spectrum (Non-Patent Document 13).

Despite the above, even now, there are a large number of G protein-coupled receptors whose ligand has not yet been identified. Twenty-seven out of such G protein-coupled receptors are presumed to have a peptide as a ligand (Non-Patent Document 14). An endogenous physiologically active peptide is present *in vivo* in a trace amount. Since an endogenous physiologically active peptide is unstable and is easily decomposed, it is not easy to identify a novel endogenous physiologically active peptide, even utilizing most-advanced science and technology.

On the other hand, since such endogenous physiologically active peptide is encoded by a gene, it has been attempted to predict it in an information science manner, using the genome sequence and the like. Specifically, using the sequence characteristics of a known endogenous physiologically active peptide and a precursor thereof as indicators, a novel endogenous physiologically active peptide sequence is predicted based on the genome sequence or the cDNA sequence. The putative peptide is prepared in a large amount by chemical synthesis, and the presence or absence of a biological activity thereof can be evaluated in various evaluation systems. As a matter of fact, novel endogenous physiologically active peptides have been identified by information science means using, as an indicator, a sequence homology with a known endogenous physiologically active peptide (Non-Patent Document 15) or an RF amide motif at the carboxyl terminus often found in such endogenous physiologically active peptide (Non-Patent Documents 16 to 18). Furthermore, novel endogenous physiologically active peptides have been identified even by methods involving a combination of multiple sequence characteristics shared by known endogenous physiologically active peptides and the precursors thereof (Non-Patent Documents 19 and 20).

However, endogenous physiologically active peptides that can be predicted by the aforementioned methods are limited to those similar to known endogenous physiologically active peptides. Thus, it is desired to develop a method for predicting a wider range of endogenous physiologically active peptides.

In general, an endogenous physiologically active peptide is encoded as a partial sequence of a precursor protein by a gene, and only specific molecules generated by cleaving the precursor protein with a prohormone convertase at specific positions have a physiological activity. Accordingly, when a novel endogenous physiologically active peptide that is not similar to known endogenous physiologically active peptides is predicted, it is extremely important to precisely predict positions at which the peptide is cleaved from the precursor. Generally, it is considered that a precursor protein is cleaved with a prohormone convertase such as PC1/3 or PC2 at the positions of two contiguous basic residues consisting of a combination of Lys and Arg (Non-Patent Documents 21 and 22). However, a large number of such two contiguous basic residues are present in proteins other than the endogenous physiologically active peptide precursor. Thus, it is extremely difficult to assume all sequences sandwiched by the aforementioned two contiguous basic residues in all target genomes of various organisms to be endogenous physiologically active peptides, and to evaluate the activities of all the sequences. Hence, an attempt to predict a sequence acting as a substrate of a prohormone convertase has also been reported (Non-Patent Document 23). However, it cannot be said that such attempt has reached a practical use level.

Thus, even now, after genome sequences have been decoded, it is extremely difficult to predict a novel physiologically active peptide.


Non-Patent Document 1: Kastin, "Handbook of biologically active peptide", Academic Press, New York, 2006
Non-Patent Document 2: Hosoda, et al., J. Pharmacol. Sci., 100, 398-410 (2006)
Non-Patent Document 3: Hinson, et al., Endocrine Reviews, 21, 138-167 (2000)
Non-Patent Document 4: Morton, et al., Nature, 4433, 289-295 (2006)
Non-Patent Document 5: Kangawa, et al., Biochem. Biophys. Res. Commun., 118, 131-139 (1984)
Non-Patent Document 6: Minamino, et al., Biochem. Biophys. Res. Commun., 130, 1078-1085 (1985)
Non-Patent Document 7: Miyata, et al., Biochem. Biophys. Res. Commun., 164, 567-574 (1989)
Non-Patent Document 8: Meunier, et al., Nature, 377, 532-535, (1995)
Non-Patent Document 9: Kojima, et al., Nature, 402, 656-660, (1999)
Non-Patent Document 10: Hinuma, et al., Nature, 393, 272-276, (1998)
Non-Patent Document 11: Tatemoto, et al., Biochem. Biophys. Res. Commun., 251, 471-476 (1998)
Non-Patent Document 12: Johnson, et al., J. Biol. Chem., 278, 52172-52178, (2003)
Non-Patent Document 13: Verdonk, et al., Assay Drug Dev. Technol., 4, 609-619, (2006)
Non-Patent Document 14: Vassilatis, et al., Proc. Natl. Acad. Sci., 100, 4903-4908 (2003)
Non-Patent Document 15: Roh, et al., J. Biol. Chem., 279, 7264-7274 (2004)
Non-Patent Document 16: Hinuma, et al., Nature Cell Biol., 400, 703-708 (2000)
Non-Patent Document 17: Chartel, et al., Proc. Natl. Acad. Sci., 100, 15247-15252 (2003)
Non-Patent Document 18: Jiang, et al., J. Biol. Chem., 278, 27652-27657 (2003)
Non-Patent Document 19: Shichiri, et al., Nat. Med., 9, 1166-1172 (2003)
Non-Patent Document 20: Mirabeau, et al., Genome Research, 17, 320-327 (2007)
Non-Patent Document 21: Seidah, et al., Brain Res., 848, 45-62 (1999)
Non-Patent Document 22: Steiner, Curr. Opin. Chem. Biol., 2, 31-39 (1998)
Non-Patent Document 23: Duckert, et al., Protein Eng. Des. Sel., 17, 107-112 (2004)


DISCLOSURE OF THE INVENTION

PROBLEMS TO BE SOLVED BY THE INVENTION

It is an object of the present invention to provide a novel endogenous physiologically active peptide.

MEANS FOR SOLVING THE PROBLEMS

As a result of intensive studies directed towards achieving the aforementioned object, the present inventors have discovered by their own method a physiologically active peptide, which had been hardly identified by conventional methods, and thus they have succeeded in obtaining a novel peptide having a hypotensive action.

In the present invention, as a gene encoding a protein, which has the structure of a secretory protein, the function of which is unknown, and which has an amino acid sequence length of less than 300 residues, a human gene (SEQ ID NO: 4) encoding a precursor protein (HC021; SEQ ID NO: 3) consisting of 100 amino acid residues, whose function is unknown, was selected from all sequences that satisfy all of the aforementioned conditions in public sequence database. The thus selected gene was cloned, and a precursor protein was produced in the form of a fusion protein of the precursor protein with *Escherichia coli* j3-galactosidase. Thereafter, the fusion protein was peptide-specifically cleaved with a prohormone convertase in a test tube. The sequence of the obtained fragment was identified using a mass spectrometer, so as to specify a position at which the precursor protein had been cleaved with the prohormone convertase. That is to say, SEQ ID NO: 5 was identified as a peptide internally cleaved when HC021 was treated with a processing enzyme (Kex2 protease derivative; Kex2-660). It was found that the aforementioned peptide is cleaved at a position between Arg at position 53 and Gly at position 54 counted from the amino terminus of HC021 (SEQ ID NO: 3). Thereafter, novel endogenous physiologically active peptides were predicted based on the position at which the precursor protein had been cleaved, and putative novel endogenous physiologically active peptide candidates were then prepared in large quantities by a chemical synthesis method. Their activities of changing the blood pressure of a rat were then measured, so that the present inventors have succeeded in finding a novel peptide exhibiting a hypotensive action.

In this method, peptide precursor candidate genes have previously specified, utilizing information science means. Thus, when this method is compared with a method comprising purifying such peptide from a tissue extract, it is able to supply large quantities of peptide samples. Moreover, since a peptide precursor protein is cleaved with a prohormone convertase by an experimental technique in this method, it makes possible to detect a specific cleavage site of the precursor protein, which is hardly predicted in an information science manner.

In the present invention, a method for measuring the hypotensive activity of a peptide is not particularly limited. Any method may be applied, as long as it is a method comprising administering the present peptide to non-human animals such as a rat or a mouse and measuring and/or evaluating a decrease in the blood pressure thereof, for example. Specifically, in addition to normal animals, hypertensive model animals such as Dahl salt-sensitive rats, Spontaneous hypertensive rats (SHR), DOCA-salt-treated rats, DOCA-salt-treated dogs, two-kidney one-clip (2K1C) Goldblatt hypertensive rats, or spinal destruction rats can be used. As a method for measuring blood pressure, an invasive blood pressure measurement method, a non-invasive blood pressure measurement method (tail cuff method), an unanesthetized unrestrained blood pressure measurement method (telemetry system), and the like can be used.

The present invention relates to a novel endogenous physiologically active peptide having a hypotensive activity or a pharmaceutically acceptable salt thereof. The present inventors have chemically synthesized a sequence comprising deletions of the amino terminus and carboxyl terminus of the novel physiologically active peptide obtained by the aforementioned method, and they have measured the hypotensive activity of the peptide. As a result, the inventors have specified that a core sequence necessary for such hypotensive activity is a sequence consisting of 9 residues LFFRRLQAY (SEQ ID NO: 23).

Moreover, the present inventors have further chemically synthesized a sequence in which the amino terminus and carboxyl terminus of the novel physiologically active peptide obtained by the aforementioned method had been extended, and sequences in which amino acids at various positions had been substituted with other amino acids. Thereafter, the inventors have measured the hypotensive activities of the thus obtained peptides. As a result, the inventors have found that such peptide maintains its hypotensive activity even if a major part of the sequence binding to the amino terminus of an active core sequence (P2) has been substituted, that such hypotensive activity is enhanced when approximately 15 residues are added to the carboxyl terminus of the active core sequence (P2), that a hydrophobic side chain in the active core sequence (P2) plays an important role for such hypotensive activity, and that such activity is maintained, even if a substitution occurs in the active core sequence (P2), if it is a conservative substitution of amino acids.

With regard to the precursor peptide (SEQ ID NO: 3) of the novel physiologically active peptide of the present invention, its amino acid sequence and/or the nucleotide sequence of DNA encoding the same are described in International Publications WO2001/072800, WO2002/002621, and WO2002/068579; US Patent Application No. US2005/0208602; Nature Biotechnology (2001), 19(5), 440-445; and Genome Research (2003), 13(10), 2265-2270. The aforementioned sequences have also been registered to the pubic sequence database such as GenBank. However, the aforementioned publications disclose only precursor polypeptides and DNA portions encoding such precursor polypeptides. They do not describe at all the novel peptide of the present invention or the fact that the peptide of the present invention has a hypotensive activity.

Furthermore, with regard to the precursor polypeptide (SEQ ID NO: 3) of the novel physiologically active peptide of the present invention, its amino acid sequence and the nucleotide sequence of DNA encoding the same, as well as 40 types of other secretory proteins, are disclosed in International Publication WO2003/052377. Regarding the activities of such proteins, it is described that a hypotensive activity can be evaluated using hypertensive rats. However, such activity is described only as one of multiple evaluation methods that may become an indicator of activity, and the publication contains no experimental data stating that all such activities are authentic. The aforementioned publication neither describes nor suggests the novel physiologically active peptide of the present invention. The publication does not even describe that a secretory protein derived from the precursor polypeptide of the novel physiologically active peptide according to the present invention has a hypotensive activity.

The present invention relates to a pharmaceutical agent comprising the peptide represented by Formula 1 or a pharmaceutically acceptable salt thereof. Since the peptide of the present invention or a pharmaceutically acceptable salt thereof has a hypotensive activity, the present invention provides a pharmaceutical composition, a therapeutic method, and a method for producing a pharmaceutical agent, in all of which the peptide represented by Formula 1 or a pharmaceutically acceptable salt thereof is used. According to the present invention, since the blood pressure of animals including humans can be lowered, it is able to treat hypertension and/or disease caused by such hypertension.

The present invention relates to an antibody prepared using the peptide of Formula 1 as an antigen and a method for measuring an endogenous hypotensive peptide using the aforementioned antibody. In addition, the present invention also includes an assay method, which comprises producing an antibody against the peptide disclosed in the present invention, and particularly an antibody against the carboxyl terminus of the aforementioned peptide, and then carrying out differential determination of the aforementioned peptide and a precursor polypeptide by utilizing the fact that the produced antibody specifically recognizes a site generated as a result of the cleavage of the aforementioned peptide from the precursor polypeptide.

Thus, the present invention includes, but not limited to, the following inventions.

(1) (i) A peptide represented by the following formula:

$$P1_n\text{-}P2\text{-}P3_m\text{-}Y \quad \text{(Formula 1)}$$

[wherein $P1_n$ represents an amino acid sequence consisting of an n number of contiguous residues starting from the carboxyl terminus of SEQ ID NO: 2, wherein n represents 0 or an integer of 1 to 21, and when n is 0, $P1_n$ does not exist;

P2 represents the amino acid sequence shown in SEQ ID NO: 23;

$P3_m$ represents an amino acid sequence consisting of an m number of contiguous residues ranging from the amino acid at position 50 from the amino terminus of SEQ ID NO: 3 to the carboxyl terminal side thereof, wherein m represents 0 or an integer of 1 to 22, and when m is 0, $P3_m$ does not exist;

the hydrogen atom of the $\alpha$-amino group of the amino acid at the amino terminus of $P1_n$ may be substituted with an acetyl group or a pyroglutamyl group; and

Y is a portion corresponding to the hydroxyl group of the $\alpha$-carboxyl group of the amino acid at the carboxyl terminus, which represents OH or $NH_2$]; or

(ii) a peptide comprising a deletion, substitution, and/or addition of one or multiple amino acids with respect to the peptide described in (i) above, and having a hypotensive activity; or

a pharmaceutically acceptable salt thereof.

(2) The peptide according to (1) above or a pharmaceutically acceptable salt thereof, wherein n is 0.

(3) The peptide according to (1) or (2) above or a pharmaceutically acceptable salt thereof, wherein n and m are 0.

(4) The peptide according to (1) above or a pharmaceutically acceptable salt thereof, wherein the peptide described in (i) above consists of an amino acid sequence selected from the group consisting of SEQ ID NOS: 1, 8, 10 to 13, 18 to 23, and 25 to 43.

(5) The peptide according to any one of (1) to (4) above or a pharmaceutically acceptable salt thereof, wherein the hydrogen atom of the $\alpha$-amino group of the amino acid at the amino terminus of $P1_n$ is substituted with an acetyl group or a pyroglutamyl group.

(6) The peptide according to any one of (1) to (5) above or a pharmaceutically acceptable salt thereof, wherein Y represents $NH_2$.

(7) A pharmaceutical composition for the treatment of hypertension or disease caused by hypertension, which comprises a peptide according to any one of (1) to (6) above or a pharmaceutically acceptable salt thereof.

(8) A method for treating hypertension or disease caused by hypertension, which comprises administering the pharmaceutical composition according to (7) above to an individual.

(9) An antibody against the peptide according to any one of (1) to (6) above.
(10) A method for determining the peptide according to any one of (1) to (6) above, which comprises detecting the peptide in a test sample using an antibody against the peptide.
(11) A method for producing the peptide according to any one of (1) to (6) above by genetic recombination technology, which comprises:

transforming a host cell with a vector containing DNA encoding the peptide;
culturing the obtained transformed cell and collecting a peptide of interest from the culture; and
carrying out a modification reaction, as necessary.

BRIEF DESCRIPTION OF THE DRAWINGS

Figure 1 is a view showing that HC021-004 and HC021-007 act to lower the blood pressure of rats. In the figure, various types of samples were administered at the points indicated with the arrows. Figure 1a shows the blood pressure before and after administration of HC021-004 (10 nmol/rat); Figure 1b shows the blood pressure before and after administration of HC021-004 (30 nmol/rat); Figure 1c shows the blood pressure before and after administration of HC021-007 (10 nmol/rat); and Figure 1d shows the blood pressure before and after administration of HC021-007 (30 nmol/rat). Figures 1e and 1f are views showing the blood pressure before and after administration of a solvent corresponding to administration of each type of peptide (10 nmol/rat or 30 nmol/rat) (which was a solvent prepared by 10 times diluting 0.1% acetic acid with a normal saline containing 0.1% bovine serum albumin (Fr.V)), respectively;
Figure 2 is a view showing the action of HC021-007 to lower the blood pressure of awake mice. The horizontal axis of the graph indicates the time (min) after administration of the peptide, and the longitudinal axis thereof indicates a change in systolic blood pressure (mmHg), using the time of administration as a standard. The symbols found in the graph indicate: a 0.1 mg/kg HC021-007 administration group (n = 3) (○); a 1 mg/kg HC021-007 administration group (n = 3) (•); a 1 mg/kg human atrial natriuretic peptide (hANP) administration group (n = 4) (△); and a normal saline administration group (n = 4) (□). The error bar in the graph indicates a standard error; and
Figure 3 is a view showing the binding of an anti- [N-Cys] -HC021-004 serum to various types of peptide sequences. Figure 3a is a view showing the ability of the aforementioned antiserum to bind to HC021-004, HC021-006, and HC021-002. The horizontal axis of the graph indicates the dilution magnification of the antiserum, and the longitudinal axis thereof indicates the absorbance at 405 nm in ELISA. The symbols found in the figure indicate: HC021-004 (•); HC021-006 (○); and HC021-002 (□). Figure 3b is a view showing the sequences of HC021-004, HC021-006, and HC021-002.

BEST MODE FOR CARRYING OUT THE INVENTION

Explanation of Terms

The term "peptide" is used in the present invention to mean a compound formed by binding a plurality of amino acids via a peptide bond. The term "amino acid" (which is also referred to as an "amino acid residue") is used herein to include native amino acids, D-/L-optical isomers thereof, and non-native amino acids. Moreover, the peptide of the present invention also includes compounds formed by modifying the amino terminus and/or carboxyl terminus of the peptide.
The term "Kex2 protease" is used in the present invention to mean a prohormone convertase (EC 3.4.21.61) of yeast. In addition, the term "Kex2-660" is used herein to mean a carboxyl terminus deletion mutant capable of efficiently secreting and expressing the Kex2 protease. Such mutant can be obtained by allowing a gene comprising a sequence ranging from Met at position 1 of the amino acid terminus of the Kex2 protease to Pro at position 660 thereof to express in yeast and then purifying it from a culture supernatant.
The term "HC021" is used in the present invention to mean a protein having the sequence shown in SEQ ID NO: 3. In the present invention, a peptide obtained from such HC021 acting as a precursor is indicated with HC021 together with hyphen and a three-digit number (for example, HC021-004, etc.).

Physiologically Active Peptide

The peptide of the present invention is a peptide represented by the following formula:

$$P1_n\text{-}P2\text{-}P3_m\text{-}Y \quad \text{(Formula 1)}$$

[wherein $P1_n$, represents an amino acid sequence consisting of an n number of contiguous residues starting from the carboxyl terminus of SEQ ID NO: 2, wherein n represents 0 or an integer of 1 to 21, and when n is 0, $P1_n$ does not exist; P2 represents the amino acid sequence shown in SEQ ID NO: 23; $P3_m$ represents an amino acid sequence consisting of an m number of contiguous residues ranging from the amino acid at position 50 from the amino terminus of SEQ ID NO: 3 to the carboxyl terminal side thereof, wherein m represents 0 or an integer of 1 to 22, and when m is 0, $P3_m$ does not exist; the hydrogen atom of the α-amino group of the amino acid at the amino terminus of $P1_n$, may be substituted with an acetyl group or a pyroglutamyl group; and Y is a portion corresponding to the hydroxyl group of the α-carboxyl group of the amino acid at the carboxyl terminus, which represents OH or $NH_{2]}$; or a peptide, which comprises a deletion, substitution, and/or addition of one or multiple amino acids with respect to the aforementioned peptide, and has a hypotensive activity; or a pharmaceutically acceptable salt thereof.

In a preferred embodiment, in the peptide of the present invention, n or m is 0 (wherein $P1_n$ or $P3_m$ does not exist). In addition, in a preferred embodiment, in the peptide of the present invention, n and m are 0 (wherein $P1_n$ and $P3_m$ do not exist). Moreover, in a preferred embodiment, the peptide of the present invention has an amino acid sequence selected from the group consisting of SEQ ID NOS: 1, 8, 10-13; 18-23, and 25-43. Furthermore, in a preferred embodiment, the amino acid terminus of $P1_n$ is substituted with an acetyl group or a pyroglutamyl group. Further, in a preferred embodiment, Y is $NH_2$.

In another preferred embodiment, an amino acid sequence comprising a deletion, substitution, and/or addition of one or several amino acids with respect to $P1_n$ consisting of 1 to 21 amino acid residues is also preferable as $P1_n$.

In a preferred embodiment, $P3_m$ represents an amino acid sequence consisting of an m number of contiguous residues ranging from the amino acid at position 50 from the amino terminus of SEQ ID NO: 3 to the carboxyl terminal side thereof, wherein m is 0 or an integer of 1 to 22. In another preferred embodiment, $P3_m$ consists of at least one amino acid selected from the group consisting of phenylalanine, lysine, glycine, and arginine. In addition, in a preferred embodiment, $P3_m$ is particularly preferably Phe (m = 1), Phe-Lys (m = 2), Phe-Lys-Gly (m = 3), or Phe-Lys-Gly-Arg (m = 4). In another preferred embodiment, an amino acid sequence comprising a deletion, substitution, and/or addition of one or several amino acids with respect to such 1 to 22 amino acid residues is also preferable as $P3_m$.

Moreover, in another preferred embodiment, an amino acid sequence comprising a deletion or addition of one or several amino acids with respect to the amino acids of $P1_n$, or an amino acid sequence comprising a substitution of 1 to an n number of amino acids, is also preferable as $P1_n$. Furthermore, an amino acid sequence comprising a conservative substitution of one out of the nine amino acids of P2 is also preferable as P2. Further, an amino acid sequence comprising a deletion or addition of one or several amino acids with respect to the amino acids of $P3_m$, or an amino acid sequence comprising a substitution of one to an m number of amino acids, is also preferable as P3.

In one embodiment, the peptide of the present invention may comprise a deletion, substitution, and/or addition of one or multiple amino acids. In addition, in one embodiment, the peptide of the present invention may comprise a deletion, substitution, and/or addition of one or several amino acids. Moreover, in one embodiment, the peptide of the present invention may comprise a deletion, substitution, and/or addition of 1, 2, 3, 4 or 5 amino acids.

<u>Method for Obtaining Peptide</u>

The peptide according to the present invention can be obtained by an ordinary method. For example, the present peptide can be produced by isolation from a naturally-occurring raw material, chemical synthesis, or recombinant DNA technology.

When the peptide of the present invention is obtained from a naturally-occurring raw material, taking into consideration the molecular weight, solubility, isoelectric point, affinity, and the like of the present peptide, the peptide can be obtained from tissues or cells that express the peptide by appropriately combining separation and purification methods, such as gel filtration, ultrafiltration, dialysis, SDS-PAGE, and various types of chromatography. When such peptide is isolated and purified from tissues or organs, in order to prevent the decomposition of the peptide of interest due to the action of protease existing in such tissues or organs, it is desired to inactivate such protease by subjecting the tissues or organs to a heat treatment in boiling water.

When the peptide of the present invention is obtained by chemical synthesis, it can be obtained by an ordinary method. Various types of methods for chemically synthesizing peptides have already been established. Thus, the peptide of the present invention can also easily be produced by a known method. For example, a classical peptide synthesis method or a solid-phase method can be applied. Specifically, the peptide of the present invention can be obtained by condensing protected amino acids according to a liquid-phase method and/or a solid-phase method, extending a peptide chain, eliminating all the protecting groups with acid, and then purifying the obtained crude product by the aforementioned purification method or the like. The peptide of the present invention can be produced by the methods described in books, for example, Chapters 2 and 3, Vol. 4, "Seikagaku Jikken Koza 1 (Biochemical Experimental Course 1), Tanpakushitsu no Kagaku (Chemistry of Proteins)," Tokyo Kagaku Dojin; or "Zoku Iyakuhin no Kaihatsu 14 (Continued Development of Pharmaceutical Products 14), Pepuchido Gosei (Peptide Synthesis)" Hirokawa Shoten.

When the peptide of the present invention is obtained by recombinant DNA technology, host cells transformed with an expression vector having DNA encoding the peptide according to the present invention are cultured, and a peptide of interest can be then collected from the culture, for example.

Examples of a vector into which a gene is to be incorporated include *Escherichia, coli* vectors (pBR322, pUC18, pUC19, etc.), *Bacillus subtilis* vectors (pUB110, pTP5, pC194, etc.), yeast vectors (YEp type, YRp type, and YIp type), and animal cell vectors (retrovirus, vaccinia virus, etc.). Any other vectors may also be used, as long as they are able to stably retain a gene of interest in host cells. Such vector is introduced into a suitable host cell. As a method for incorporating a gene of interest into a plasmid or a method for introducing such gene of interest into a host cell, the method described in Molecular Cloning (Sambrook et al., 1989) may be used, for example.

In order to allow a peptide gene of interest to express in the aforementioned plasmid, a promoter is allowed to be connected upstream of the gene, such that the promoter functions. As a promoter used in the present invention, any type of promoter may be used, as long as it is a suitable promoter corresponding to a host cell used in the expression of the gene of interest. When a host cell to be transformed is genus Escherichia, a lac promoter, a trp promoter, a Ipp promoter, a λPL promoter, a recA promoter, and the like may be used, for example. When a host cell to be transformed is genus Bacillus, an SPO1 promoter, an SPO2 promoter, a penP promoter, and the like may be used. When such host cell is yeast, a GAP promoter, a PHO5 promoter, an ADH promoter, and the like may be used. When such host cell is an animal cell, an SV40 promoter, a CMV promoter, a retrovirus-derived promoter, and the like may be used.

When host cells are transformed with the thus obtained vector containing a gene of interest, examples of such host cells that can be used herein include bacteria (for example, genus Escherichia, genus Bacillus, etc.), yeasts (genus Saccharomyces, genus Pichia, genus Candida, etc.), and animal cells (CHO cells, COS cells, etc.). As a medium used in culture, a liquid medium is appropriate, and such liquid medium particularly preferably contains a carbon source, a nitrogen source, and the like, which are necessary for the growth of the transformed cells to be cultured therein. It is also possible to add to such medium vitamins, growth promoting factors, serum, and the like, if desired.

The peptide of the present invention can be directly produced, using, as a host, a cell having a processing protease activity of cleaving a polypeptide as a precursor at a suitable position. Furthermore, if a cell having a peptidylglycine-α-amidating enzyme activity as well as a processing protease activity is used as a host, the peptide of the present invention having an amidated carboxyl terminus can be directly produced. Such host cell having a processing protease activity and a peptidylglycine-α-amidating enzyme activity can be selected by transforming host cells with an expression vector containing DNA encoding the precursor polypeptide and then confirming that the transformed cells produce a peptide having a hypotensive activity.

Moreover, the peptide of the present invention can also be produced by producing a precursor polypeptide from a host cell and then obtaining a peptide of interest from the precursor polypeptide in a test tube. As a DNA sequence encoding such precursor polypeptide to be used in transformation, there may be used either the cDNA sequence of a gene encoding a peptide of interest, or a DNA sequence encoding polypeptide in which the peptides sandwiching a processing protease cleavage sequence are aligned in tandem. For example, host cells are transformed with a vector containing DNA encoding a precursor polypeptide and are then cultured, and then, the precursor polypeptide is extracted and purified from a cultured cell mass, cells, or a culture solution. Thereafter, a purified processing protease such as Kex2-660 is allowed to react with the precursor polypeptide, so as to cleave it, and a purified carboxypeptidase or peptidylglycine-α-amidating enzyme is allowed to react with the precursor polypeptide, as necessary, so as to modify the carboxyl terminus thereof, thereby obtaining a peptide of interest. Herein, in order to purify the peptide of the present invention from a culture solution or reaction solution containing the peptide of the present invention, the same separation and purification method as in the case of obtaining a naturally-occurring peptide may be applied.

For example, *Escherichia coli* is transformed with a vector containing DNA encoding a polypeptide formed by fusing, via a linker portion capable of being cleaved with a processing enzyme (for example, Kex2 protease), a sequence obtained by removing a secretory signal sequence (a portion ranging from the amino terminus of SEQ ID NO: 3 to the amino acid at position 21) from the amino acid sequence (SEQ ID NO: 3) of HC021 with a carboxyl terminus having a sequence ranging from the N terminus of *Escherichia coli* β-galactosidase to the residue at position 139 (wherein the cysteine residues at positions 77 and 123 counted from the N terminus have been substituted with serine residues). Thereafter, the obtained fusion protein is treated with a processing enzyme (for example, Kex2-660 as a Kex2 protease derivative), so as to obtain a peptide having, at the carboxyl terminus thereof, a sequence prepared by adding Phe-Lys-Gly-Arg to the carboxyl terminus of SEQ ID NO: 23.

Furthermore, a technique of eliminating the Arg at the carboxyl terminus by allowing the Arg to react with carboxypeptidase capable of releasing basic amino acids (for example, carboxypeptidase-B) in a test tube has been publicly known. Thus, the aforementioned peptide is treated with carboxypeptidase, so as to obtain a peptide having, at the carboxyl terminus thereof, a sequence prepared by adding Phe-Lys-Gly to the carboxyl terminus of SEQ ID NO: 23.

Still further, a technique of converting the Gly at the carboxyl terminus to amide by allowing the Gly to react with a peptidylglycine-α-amidating enzyme in a test tube has been publicly known. Thus, after the aforementioned peptide has been treated with carboxypeptidase, it is further treated with such peptidylglycine-α-amidating enzyme, so as to obtain a peptide having, at the carboxyl terminus thereof, a sequence prepared by adding Phe-Lys-amide to the carboxyl terminus of SEX ID NO: 23.

## Pharmaceutically Acceptable Salt

In addition to the peptide of Formula 1, the present invention also includes a pharmaceutically acceptable salt thereof. Examples of such pharmaceutically acceptable salt include a salt with an inorganic base, a salt with an organic base, a salt with an inorganic acid, a salt with an organic acid, and a salt with a basic or acidic amino acid. Of these, a sodium salt and a potassium salt are most preferable in the present invention.

Preferred examples of a salt with an inorganic base include: alkaline metal salts such as a sodium salt and a potassium salt; alkaline-earth metal salts such as a calcium salt and a magnesium salt; and an aluminum salt and an ammonium salt. Preferred examples of a salt with an organic base include salts with trimethylamine, triethylamine, pyridine, picoline, ethanolamine, diethanolamine, triethanolamine, dicyclohexylamine, N,N'-dibenzylethylenediamine, or the like.

Preferred examples of a salt with an inorganic acid include salts with hydrochloric acid, hydrobromic acid, nitric acid, sulfuric acid, phosphoric acid, or the like. Preferred examples of a salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, or the like.

Preferred examples of a salt with a basic amino acid include salts with arginine, lysine, ornithine, or the like. Preferred examples of a salt with an acidic amino acid include aspartic acid, glutamic acid, or the like.

## Pharmaceutical Agent

The peptide of the present invention or a pharmaceutically acceptable salt thereof has a hypotensive action. In addition, the peptide of the present invention or a pharmaceutically acceptable salt thereof may be used for animals including humans, directly or by mixing with a known pharmaceutically acceptable carrier, excipient, extender, or the like.

The dosage form is not particularly limited. When the peptide of the present invention or a pharmaceutically acceptable salt thereof is administered to an adult via intravenous injection, the dose per administration is 0.8 μg to 8 mg/kg, preferably 4 μg to 2 mg/kg, and more preferably 8 μg to 0.8 mg/kg. It is desired to administer such dose 1 to 3 times per day.

The peptide of the present invention or a pharmaceutically acceptable salt thereof may be administered orally or parenterally in the form of a solid preparation such as a tablet, a capsule, a granule or a powder; or in the form of a liquid preparation such as a syrup or an injection. Moreover, as necessary, pharmaceutical additives such as an antiseptic, an antioxidant, a coloring agent and a sweetener may also be used.

The peptide of the present invention or a pharmaceutically acceptable salt thereof may be used in combination with a pharmaceutically acceptable carrier. As such pharmaceutically acceptable carrier, various types of organic or inorganic carrier substances, which are commonly used as pharmaceutical materials, are used. Such carrier substance is mixed as an excipient, a lubricant, a binder, or a disintegrator into a solid preparation; and as a solubilizer, a suspending agent, an isotonizing agent, a buffer, a soothing agent, or the like into a liquid preparation.

Preferred examples of an excipient include lactose, sucrose, D-mannitol, starch, crystalline cellulose, and light anhydrous silicic acid. Preferred examples of a lubricant include magnesium stearate, calcium stearate, talc, and colloidal silica.

Preferred examples of a binder include crystalline cellulose, sucrose, D-mannitol, dextrin, hydroxypropylcellulose, hydroxypropylcellulose, and polyvinylpyrrolidone.

Preferred examples of a disintegrator include starch, carboxymethylcellulose, carboxymethylcellulose calcium, croscarmellose sodium, and carboxymethyl starch sodium.

Preferred examples of a solvent include water for injection, alcohol, propylene glycol, macrogol, sesame oil, and corn oil.

Preferred examples of a solubilizer include polyethylene glycol, propylene glycol, D-mannitol, benzyl benzoate, ethanol, trisaminomethane, cholesterol, triethanolamine, sodium carbonate, and sodium citrate.

Preferred examples of a suspending agent include: surfactants such as stearyl triethanolamine, sodium lauryl sulfate, lauryl aminopropionate, lecithin, benzalkonium chloride, benzethonium chloride, and glycerin monostearate; and hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose.

Preferred examples of an isotonizing agent include sodium chloride, glycerin, and D-mannitol.
Preferred examples of a buffer include phosphate, acetate, carbonate, and citrate.
A preferred example of a soothing agent is benzyl alcohol.
Preferred examples of an antiseptic include paraoxybenzoic acid esters, chlorobutanol, benzyl alcohol, phenethyl alcohol, dehydroacetic acid, and sorbic acid.
Preferred examples of an antioxidant include sulfite and ascorbic acid.

Antibody and Assay

From a certain viewpoint, the present invention relates to an antibody against the peptide of Formula 1. An antibody whose antigen is the peptide of Formula 1 having a hypotensive activity can be obtained by a known method. The antibody of the present invention may be either a monoclonal antibody or a polyclonal antibody.
In addition, from another viewpoint, the present invention relates to a method for measuring a peptide having a hypotensive activity, using such antibody.
In a preferred embodiment, the antibody of the present invention recognizes the peptide of Formula 1 according to the present invention, but it does not recognize a precursor peptide thereof. Using such antibody, the novel peptide of the present invention can be selectively purified and determined.
Specific embodiments of the aforementioned assay method will be described below. However, the assay method is not limited thereto.
Specifically, examples of the aforementioned assay method include:

(i) an assay method for determining the peptide or the like according to the present invention, which comprises allowing an antibody against the peptide of the present invention to competitively react with a test substance and the labeled peptide of the present invention contained in a test sample, and then measuring the ratio of the labeled peptide of the present invention or the like bound to the antibody; and
(ii) an assay method for determining the peptide or the like of the present invention, which comprises allowing a test sample to simultaneously or continuously react with the antibody of the present invention insolubilized on a carrier and another labeled antibody of the present invention, and then measuring the activity of a labeling agent on such insolubilized carrier or/and the activity of a labeling agent that has not been captured on such insolubilized carrier. In the aforementioned assay method, one of the two antibodies is preferably an antibody that recognizes the peptide according to the present invention, but does not recognize a precursor polypeptide.

Moreover, as an assay method for the peptide or the like of the present invention, determination of the peptide or the like of the present invention may be carried out using an antibody against the aforementioned peptide. Further, detection involving tissue staining and the like may also be carried out.
For such purposes, an antibody molecule may be used as is. Otherwise, an F(ab')2, Fab', or Fab fraction of such antibody molecule may also be used.
The type of an assay method for determining the peptide or the like of the present invention using the aforementioned antibody is not particularly limited. Any type of measurement method may be used, as long as it is a measurement method, which comprises detecting the amount of an antibody, an antigen, or an antibody-antigen complex that corresponds to the amount of an antigen (for example, the amount of a peptide) in a test sample by chemical or physical means, and calculating the aforementioned amount based on a standard curve prepared using a standard solution containing a known amount of antigen. For example, nephrometry, a competition method, an immunometric method, and a sandwich method are preferably used. In terms of sensitivity and specificity, a sandwich method is particularly preferably used.
Among the assay methods according to the present invention, examples of a labeling agent used in the measurement method of the present invention using a labeling substance include a radioisotope, an enzyme, a fluorescent substance, and a luminescent substance. Examples of a radioisotope that can be used in the present invention include 125I, 131I, 3H, and 14C. In addition, as the aforementioned enzyme, an enzyme that is stable and has a large specific activity is preferable. For example, β-galaetosidase, β-glucosidase, alkaline phosphatase, peroxidase, and malate dehydrogenase may be used. Moreover, examples of a fluorescent substance include fluorescamine and fluorescein isocyanate. Examples of a luminescent substance include luminol, a luminol derivative, luciferase, and lucigenin. Furthermore, a biotin-avidin system may also be used in the binding of an antibody or an antigen to a labeling agent.

EXAMPLES

The present invention will be more specifically described in the following examples. However, the following specific examples are not intended to limit the technical scope of the present invention.

The meanings of the major abbreviations used in the present examples are indicated below.
HBTU: N-[(1H-benzotriazol-1-yl(dimethylamino)methylene)-N-methylmethanaminium hexafluorophosphate N-oxide
HOBt: 1-hydroxybenzotriazole
Trt: trityl
Pmc: 2,2,5,'7,8-pentamethylchroman-6-sullonyl
OtBu: t-butyl ester
Mtt: 4-methyltrityl
TIPS: triisopropylsilane

Example 1: Chemical synthesis of HC021 peptides

Taking into consideration the information that HC021 is cleaved with Hex2-660 at a position between the Arg at position 53 and the Gly at position 54 and the mechanism whereby an endogenous physiologically active peptide precursor is generally processed in a cell so as to cause the biosynthesis of a peptide (wherein two or more contiguous basic amino acid residues are cleaved with a prohormone convertase on the carboxyl terminal side; the basic amino acid at the carboxyl terminus is removed with carboxyl peptidase; and the Gly at the carboxyl terminus is amidated with a peptidylglycine-α-amidating enzyme), the following peptide sequences were created, and chemical synthesis was then carried out.

HC021-001 ATVRNEDKWKPLNNPRNRDLFF (SEQ ID NO: 6)
HC021-002 LQAYFKGRGLDLGTFPNPFPTNENP (SEQ ID NO: 7)
HC021-004 NRDLFFRRLQAYFK-amide (SEQ ID NO: 8)
HC021-006 LQAYFK-amide (SEQ ID NO: 9)
HC021-007 ATVRNEDKWKPLNNPRNRDLFFRRLQAYFK-amide (SEQ ID NO: 10)

A peptide chain was extended mainly using a peptide synthesizer (433A; manufactured by Applied Biosystems), and a protected peptide derivative-resin was constructed by an Fmoc method. The obtained protected peptide resin was deprotected with trifluoroacetic acid (TFA) or diluted TFA containing various scavengers, and a released peptide was then purified. The released peptide was purified by reverse phase HPLC using a C18 column. At the same time, its purity was measured, and its structure was confirmed by mass spectrometry.

The peptide of the present invention can be produced by a common peptide synthesis method. As a typical synthesis example, the synthesis of HC021-004 will be described below.

Synthesis of HC021-004

For the synthesis, the peptide synthesizer 433A manufactured by Applied Biosystems was used. An Fmoc-Amide resin (373 mg; 0.25 nmol) was placed in a reaction vessel, and a FastMoc0.25 program was then applied using HBTU/HOBt as a condensing agent. Double coupling was carried out, as necessary, so as to construct a peptide resin. Finally, the Fmoc group at the N-terminus was eliminated by treatment with piperidine. The peptide resin was dried, and it was then treated with 15 ml of a deprotecting reagent consisting of TFA : phenol : water : TIPS = 8.8 : 0.5 : 0.5 : 0.2 (v/v) for 2 hours. After completion of the reaction, the resultant was filtrated to remove the resin, and the remaining solution was then concentrated under a reduced pressure. Thereafter, ether was added to the residue for precipitation, and the precipitate was then collected by filtration, thereby obtaining 352.6 mg of a crude peptide. 176.3 mg (half amount of the above) of the obtained peptide was added to a YMC ODS column (5 μm, 2 cm × 25 μm), and elution was then carried out in 0.1% trifluoroacetic acid in a linear gradient manner of an acetonitrile concentration from 0% to 60% for 60 minutes (flow rate: 10 mL/min). The remaining crude peptide was also purified with the YMC ODS column, and a fraction with high purity obtained from the two times of chromatography was freeze-dried, so as to obtain 267.7 mg of powders. The powders were added to a YMC Protein-RP column (5 μm C4, 2 cm × 25 cm), and elution was then carried out in 0.1% trifluoroacetic acid in a linear gradient manner of an acetonitrile concentration from 0% to 60% for 60 minutes (flow rate: 10 mL/min). After completion of freeze-drying, 245 mg of peptide of interest was obtained. ESI-MS measurement value: 1873.0 (theoretical value: 1873.17)

Example 2: Effects of peptides on blood pressure

50 mg/kg pentobarbital sodium was intraperitoneally administered to 8- to 10-week-old Sprague-Dawley IGS male rats (Charles River Laboratories Japan, Inc.) for anesthesia. Thereafter, a tracheal cannula (PE 250), a femoral vein cannula (PE50), and a femoral artery cannula (PE50) were inserted into each rat. The femoral artery cannula was connected with a pressure transducer (PE23XL; manufactured by Nihon Kohden Corporation), and an apparatus formed by incorporating a blood pressure measurement unit (AP-641G; manufactured by Nihon Kohden Corporation) and an instantaneous heart rate measurement unit (AT-601G; manufactured by Nihon Kohden Corporation) into a multi-purpose preamplifier (RP-6004; manufactured by Nihon Kohden Corporation) was used to record the blood pressure and heart rate of each rat. The blood pressure was calibrated using a mercury manometer included in a stand-type electromanometer (MP-25S; manufactured by Nihon Kohden Corporation). The recording paper was adjusted, such that 10 mm or 20 mm on the recording paper corresponded to 100 mmHg. Acetylcholine (10 μg/rat) was used as a positive control in blood pressure fluctuation, and a solvent in which each peptide had been dissolved was used as a negative control in blood pressure fluctuation.

Each peptide was dissolved in a concentration of 1 mM or 10 mM in a 0.1% acetic acid solution to prepare a peptide solution. Thereafter, depending on dose, it was diluted with a normal saline containing 0.1% bovine serum albumin (Fr.V). In the case of the dose of 10 nmol/rat, a 1 mM peptide solution was 10 times diluted, and 100 μL of the diluted solution was administered. In the case of the dose of 30 nmol/rat, a 1 mM peptide solution was 10 times diluted, and 300 μL of the diluted solution was administered. In the case of the dose of 100 nmol/rat, a 10mM peptide solution was 10 times diluted, and 100 μL of the diluted solution was administered. Each peptide was intravenously administered via the femoral vein cannula, and the systolic blood pressure, diastolic blood pressure, and heart rate were then measured.

The results are shown in Table 1 and Figure 1. HC021-004 and HC021-007 were observed to have a hypotensive activity.

[Table 1]

Table 1: Activities of HC021 derivative peptides

| Compound No. | Compound structure | SEQ ID NO: | Hypotensive activity (dose/rat) | | |
|---|---|---|---|---|---|
| | | | 10 nmol | 30 nmol | 100 nmol |
| 1 | ATVRNEDKWKPLNNPRNRDLFF (HC021-001) | 6 | - | - | - |
| 2 | LQAYFKGRGLDLGTFPNPFPTNENP (HC021-002) | 7 | - | - | - |
| 3 | NRDLFFRRLQAYFK-amide (HC021-004) | 8 | + | ++ | +++ |
| 4 | LQA YFK-amide (HC021-006) | 9 | NT | - | - |
| 5 | ATVRNEDKWKPLNNPRNRDLFFRRLQAYFK-amide (HC021-007) | 10 | ++ | +++ | NT |
| NT: not tested | | | | | |

Example 3: Search for active regions of HC021 peptides

Various HC021 derivative peptides were synthesized in accordance with Example 1, and such peptide was then administered to each rat at a dose of 10 nmol/rat, 30 nmol/rat, or 100 nmol/rat by the method described in Example 2. Thereafter, the hypotensive activity of each derivative peptide was measured. With regard to the hypotensive activity of each derivative peptide, when a certain peptide acted on two or more rats, the peptide was considered to have the activity. The strength of such activity was determined using a median of activity in repeated experiments. In addition, in order to make a comparison among the activities of various derivative peptides, the strength of hypotensive activity was defined as follows.

Strength of Hypotensive Activity

- Extremely strong activity (+++): A decrease in systolic blood pressure is 30 mmHg or more, and the time required for recovery is 5 minutes or more.
- Strong activity (++): A decrease in systolic blood pressure is 20 mmHg or more and less than 30 mmHg, or the time required for recovery is less than 5 minutes.
- Activity observed (+): A decrease in systolic blood pressure is less than 20 mmHg.
- Activity not observed (-)
  The results are shown in Table 2 below.

Compounds 3 (HC021-004), 5 (HC021-007), 12, 14, 16, 17, and 19 enhanced their hypotensive activity in a dose-dependent manner.

From the results of compounds 1 to 26, it was found that 9 residues (LFFRRLQAY: SEQ ID NO: 23) existing on the carboxyl terminal side of each compound (hereinafter referred to as an "active core sequence") are important for the activity. From the results of compounds 3, 5-10, and 15-17, it was found that although the amino terminus of such active core sequence is extended, its activity is maintained. From the results of compounds 3,11-15, 18, 19, 24, 25, and 27-30, it was found that although the carboxyl terminus of the active core sequence is extended by several residues, its activity is maintained, and that if the carboxyl terminus is further extended, its activity is enhanced. Moreover, from the results of compounds 3, 5, 8-15, 18, and 19, it was found that the amide structure of the carboxyl terminus enhances its activity, although it is not essential for the expression of the activity. From the results of compounds 31-33, it was found that the modification of the amino group at the amino terminus does not influence on the activity.

[Table 2]

Table 2: Activities of HC021 peptide-extended and -reduced products

| Compound No. | Compound structure | | SEQ ID NO: | Hypotensive activity (dose/rat) | | |
|---|---|---|---|---|---|---|
| | | | | 10 nmol | 30 nmol | 100 nmol |
| 6 | NGATVRNEDKWKPLNNPRNRDLFFRRLQAYFK-amide | | 20 | - | + | NT |
| 7 | GATVRNEDKWKPLNNPRNRDLFFRRLQAYFK-amide | | 19 | - | ++ | NT |
| 8 | ATVRNEDKWKPLNNPRNRDLFFRRLQAYFK | | 10 | NT | - | +++ |
| 5 | ATVRNEDKWKPLNNPRNRDLFFRRLQAYFK-amide (HC021-007) | | 10 | ++ | +++ | NT |
| 9 | ATVRNEDKWKPLNNPRNRDLFFRRLQAYF | | 18 | NT | - | +++ |
| 10 | ATVRNEDKWKPLNNPRNRDLFFRRLQAYF-amide | | 18 | - | ++ | NT |
| 1 | ATVRNEDKWKPLNNPRNRDLFF(HC021-001) | | 6 | - | - | - |
| 11 | | NRDLFFRRLQAYFKG | 21 | - | - | + |
| 12 | | NRDLFFRRLQAYFKG-amide | 21 | - | ++ | +++ |
| 13 | | NRDLFFRRLQAYPKGR | 22 | NT | - | ++ |
| 14 | | NRDLFFRRLOAYFKGR-amide | 22 | + | ++ | NT |
| 15 | | NRDLFFRRLQAYFK | 8 | - | - | + |
| 3 | (HC021-004) | NRDLFFRRLQAYFK-amide | 8 | + | ++ | +++ |
| 16 | | RDLFFRRLQAYFK-amide | 11 | - | ++ | +++ |
| 17 | | DLFFRRLQAYFK-amide | 12 | - | ++ | +++ |
| 18 | | LFFRRLQAYFK | 13 | NT | - | ++ |
| 19 | | LFFRRLQAYFK-amide | 13 | + | ++ | +++ |
| 20 | | FFRRLQAYFK-amide | 14 | NT | - | - |
| 21 | | FRRLQAYFK-amide | 15 | NT | - | - |
| 22 | | RRLQAYFK-amide | 16 | NT | - | - |
| 23 | | RLQAYFK-amide | 17 | NT | - | - |
| 4 | (HC021-006) | LQAYFK-amide | 9 | NT | - | - |
| 24 | | LFFRRLQAYF-amide | 1 | NT | ++ | NT |
| 25 | | LFFRRLQAY-amide | 23 | NT | - | + |

(continued)

| Compound No. | Compound structure | SEQ ID NO: | Hypotensive activity (dose/rat) | | |
|---|---|---|---|---|---|
| | | | 10 nmol | 30 nmol | 100 nmol |
| 26 | LFFRRLQA-amide | 24 | NT | - | - |
| 27 | LFFRRLQAYFKGRGLDLGTFPNPF | 25 | +++ | NT | NT |
| 28 | LFFRRLOAYFKGRGLDLGTFPNPF-amide | 25 | +++ | NT | NT |
| 29 | LFFRRLQAYFKGRGLDLGTFPNPFPTNENPR | 26 | +++ | NT | NT |
| 30 | LFFRRLOAYFKGRGLDLGTFPNPFPT-NENPR-amide | 26 | +++ | NT | NT |
| 31 | acetyl-ATVRNEDEWKPLNNPRNRDLFFRRLQAYFK-amide | 10 | NT | +++ | NT |
| 32 | acetyl-NRDLFFRRLQAYFK-amide | 8 | NT | ++ | NT |
| 33 | pyr-EDKWKPLNNPRNRDLFFRRLQAYFK-amide | 27 | ++ | NT | NT |
| NT: not tested; underlined portion: core sequence; pyr-: pyroglutamyl group | | | | | |

Example 4: Influence of sequence substitution of HC021 peptides on activity

Various HC021 peptide sequence-substituted products were synthesized in accordance with Example 1, and their hypotensive activity was then measured according to the method of Example 3.

The results are shown in the following Table 3. The definition of the strength of hypotensive activity was the same as that of Example 3.

From the results of Compounds 34-40, it was found that, even if a major part of the sequence on the amino terminal side binding to the active core sequence is substituted, it maintains the activity. In addition, from the results of compounds 48 and 49, it was found that, even if a major part of the sequence on the carboxyl terminal side binding to the active core sequence is substituted, it maintains the activity. Moreover, from the results of compounds 36-38 and 43 (conservative substitution of a hydrophilic side chain) and compounds 44-47 (conservative substitution of hydrophobic/aromatic side chain), it was found that, even if a conservative substitution of approximately 1 residue occurs in the active core sequence is substituted, it maintains the activity. From the results of compounds 46 and 47, it was found a hydrophobic side chain is important for the activity in the active core sequence.

[Table 3]

Table 3: Activities of HC021 peptide sequence-substituted products

| Compound No. | Compound structure | SEQ ID NO: | Hypotensive activity (dose/rat) | | |
|---|---|---|---|---|---|
| | | | 10 nmol | 30 nmol | 100 nmol |
| 5 | ATVRNEDKWKPLNNPRNRDLFFRRLQAYFK-amide (HC021-007) | 10 | ++ | +++ | NT |
| 34 | ATLRNEDTWKPLSNPRNRDLFFRRLQAYFK-amide | 28 | - | + | NT |
| 35 | ATLGSEDTWKPLSNPRNRDLFFRRLQAYFK-amide | 29 | - | ++ | NT |
| 36 | ATLRNEDTWKPLSNPRNRELFFRSLQAYFK-amide | 30 | - | - | +++ |
| 37 | ATLGSEDTWKPLSNPRNRELFFRSLQAYFK-amide | 31 | - | - | ++ |
| 38 | ATLGSEDTWKPLSNPRNRELFFRSQAYF-amide | 32 | - | - | ++ |
| 39 | FVPIFTYGELQRMQNRDLFFRRLQAYFK-amide | 33 | - | - | ++ |

(continued)

| Compound No. | Compound structure | SEQ ID NO: | Hypotensive activity (dose/rat) | | |
|---|---|---|---|---|---|
| | | | 10 nmol | 30 nmol | 100 nmol |
| 40 | VQQRKESKKPPAKLQPRNRDLFFRRLQAYFK-amíide | 34 | NT | ++ | NT |
| 3 | (HC021-004) NRDLFFRRLQAYFK-amide | 8 | + | ++ | +++ |
| 41 | NRELFPRRLQAYFK-amide | 35 | - | ++ | NT |
| 42 | NRDLFFRRLQAYAK-amide | 36 | - | - | ++ |
| 19 | LFFRRLQAYFK-amide | 13 | + | ++ | +++ |
| 43 | LFFRSLQAYFK-amide | 37 | - | ++ | NT |
| 44 | LPFRRLQALFK-amide | 38 | - | ++ | NT |
| 45 | LFFRRLQA(Cha)FK-amide | 39 | + | +++ | NT |
| 46 | LFFRRLQAWFK-amide | 40 | - | - | + |
| 47 | LFFRRLQAHFK-amide | 41 | - | - | +++ |
| 30 | LFFRRLQAYFKGRGLDLGTFPNPFPTNENPR-amide | 26 | +++ | NT | NT |
| 48 | LFFRRLQAYFKNGAEDESAEAFPLEF-amide | 42 | +++ | NT | NT |
| 49 | LFFRRLQAYFKASASADYEEQKNSFHNYLK-amide | 43 | +++ | NT | NT |
| NT: not tested; underlined portion: amino acid residues substituted from HC021; Cha: cyclohexylalanine | | | | | |

Example 5: Hypotensive activity on awake rats

50 mg/kg pentobarbital sodium was intraperitoneally administered to 8- to 10-week-old Sprague-Dawley IGS male rats (Charles River Laboratories Japan, Inc.) for anesthesia. Thereafter, a jugular vein cannula (Silascon 0.5-1.0) was inserted into each rat for use in peptide administration, and a carotid artery cannula (PE50) was inserted into each rat for use in the measurement of blood pressure. At least 3 hours after the surgery, it was confirmed that the rats were awoken from the anesthesia based on general conditions such as the posture, respiration, and the like of the rats. Thereafter, the rats were subjected to the experiment.

The carotid artery cannula was connected with a pressure transducer (PE23XL; manufactured by Nihon Kohden Corporation), and an apparatus forme by incorporating a blood pressure measurement unit (AP-641G; manufactured by Nihon Kohden Corporation) and an instantaneous heart rate measurement unit (AT-601G; manufactured by Nihon Kohden Corporation) into a multi-purpose preamplifier (RP-6004; manufactured by Nihon Kohden Corporation) was used to record the blood pressure and heart rate of each rat. The blood pressure was calibrated using a mercury manometer included in a stand-type electromanometer (MP-25S; manufactured by Nihon Kohden Corporation). The recording paper was adjusted, such that 20 mm or 40 mm on the recording paper corresponded to 100 mmHg. A solvent in which each peptide had been dissolved was used as a negative control in blood pressure fluctuation.

Each peptide was dissolved in a concentration of 1 mM in a 5% mannitol solution to prepare a peptide solution. Thereafter, depending on dose, it was diluted with a 5% mannitol solution. In the case of the dose of 10 nmol/rat, a 1 mM peptide solution was 30 times diluted, and 300 μL of the diluted solution was administered. In the case of the dose of 30 nmol/rat, a 1mM peptide solution was 10 times diluted, and 300 μL of the diluted solution was administered. In the case of the dose of 100 nmol/rat, a 1mM peptide solution was 3 times diluted, and 300 μL of the diluted solution was administered. Each peptide was intravenously administered via the jugular vein cannula, and the systolic blood pressure, diastolic blood pressure, and heart rate were then measured.

The results are shown in Table 4. The definition of the strength of hypotensive activity was the same as that of Example 3.

As in the case of using the anesthetized rats of Examples 2, 3, and 4, HC021-004, HC021-007, and compound 36 were observed to have a hypotensive activity even on the awake rats.

[Table 4]

Table 4: Activities of HC021 peptides on awake rats

| Compound No. | Compound structure | SEQ ID NO: | Hypotensive activity (dose/rat) | | |
|---|---|---|---|---|---|
| | | | 10 nmol | 30 nmol | 100 nmol |
| 3 | NRDLFFRRLQAYFK-amide (HC021-004) | 8 | - | - | ++ |
| 5 | ATVRNEDKWKPLNNPRNRDLFFTRRLQAYFK-amide (HC021-007) | 10 | - | ++ | +++ |
| 36 | ATLRNEDTWKPLSNPRNRELFFRSLQAYFK-amide | 30 | - | - | ++ |

Example 6: Hypotensive activity on awake mice

A C57BL/6 mice with a body weight of 30 to 40 g (Charles River Laboratories Japan, Inc.) were each placed in a retaining appliance of a non-preheating, non-invasive blood pressure monitor for mice and rats (MODEL MK-2000; Muromachi Kikai Co., Ltd.), and a cuff-pulse sensor was then attached to the root of the tail. After confirming that the blood pressure of each mouse had been stable, the mouse was subjected to the experiment.

A peptide solution was prepared by diluting each peptide with a normal saline, depending on the dose. In the case of the dose of 0.1 mg/kg, a 0.02 mg/mL peptide solution was prepared. In the case of the dose of 1 mg/kg, a 0.2 mg/mL peptide solution was prepared. The amount of the solution to be administered was set at 5 mL/kg. As a positive control, a human atrial natriuretic peptide (hANP) was used. As a negative control, a normal saline was used. Each peptide was administered via the caudal vein using a syringe with a 29G needle. Three and ten minutes after the administration, the systolic blood pressure and the heart rate were measured.

The results are shown in Figure 2.

As in the case of using the anesthetized rats of Examples 2, 3, and 4, HC021-007 exhibited a hypotensive activity even on the awake mice.

Example 7: Production of antibody against peptides

Using [N-Cys]-HC021-004 (a peptide prepared by binding cysteine to the amino terminus of HC021-004) as an antigen, a rabbit was immunized with the antigen to produce antiserum (anti-[N-Cys]-HC021-004 serum).

The ability of this antiserum to bind to peptides having various types of sequences was examined using Protein DetectorTM ELISA kit (AP; BluePhos System; manufactured by KPL). Reactions were all carried out at a room temperature. A 96-well immunoplate (maxi-coated; manufactured by Nunc) was coated (amount: 100 uL/well) with each peptide having a different type of sequence (concentration: 1 ng/100 uL) for 1 hour. Then, it was blocked with a 300 uL/well blocking solution for 1 hour. Thereafter, the serially diluted antiserum was added in an amount of 100 uL/well to the immunoplate, followed by reaction for 1 hour. After completion of washing, 500-times diluted Anti-rabbit IgG HRP Ab (manufactured by KPL) was added thereto in an amount of 100 uL/well, followed by reaction for 1 hour. After completion of washing, ABTS (manufactured by KPL) used as a substrate was added thereto in an amount of 100 uL/well. Thirty minutes later, the absorbance at 405 nm was measured.

The results are shown in Figure 3. The antiserum bound to both HC021-004 and HC021-006 at an almost equivalent level. However, the antiserum did not bind to HC021-002 at all. As a result, it was considered that the antiserum did not recognize HC021 as a precursor sequence, and that it recognized a peptide having an active core sequence on the carboxyl terminal side.

Using the antibody against the peptide of the invention of the present application, a precursor polypeptide can be differentiated, and the peptide of the present invention can be determined.

INDUSTRIAL APPLICABILITY

The present invention relates to a novel physiologically active peptide. When the peptide of the present invention or a pharmaceutically acceptable salt thereof is administered to a human or another animal, it decreases the blood pressure thereof. Accordingly, it is useful as a pharmaceutical agent for improving disease caused by hypertension. Moreover, an antibody against the peptide of the present invention is useful for the diagnosis of the disease.

## Claims

**1.** (i) A peptide represented by the following formula:

$$P1_n\text{-}P2\text{-}P3_m\text{-}Y \quad \text{(Formula 1)}$$

[wherein $P1_n$ represents an amino acid sequence consisting of an n number of contiguous residues starting from the carboxyl terminus of SEQ ID NO: 2, wherein n represents 0 or an integer of 1 to 21, and when n is 0, $P1_n$ does not exist;

P2 represents the amino acid sequence shown in SEQ ID NO: 23;
$P3_m$ represents an amino acid sequence consisting of an m number of contiguous residues ranging from the amino acid at position 50 from the amino terminus of SEQ ID NO: 3 to the carboxyl terminal side thereof, wherein m represents 0 or an integer of 1 to 22, and when m is 0, $P3_m$ does not exist;
the hydrogen atom of the α-amino group of the amino acid at the amino terminus of $P1_n$ may be substituted with an acetyl group or a pyroglutamyl group; and
Y is a portion corresponding to the hydroxyl group of the α-carboxyl group of the amino acid at the carboxyl terminus, which represents OH or $NH_2$]; or
(ii) a peptide comprising a deletion, substitution, and/or addition of one or multiple amino acids with respect to the peptide described in (i) above, and having a hypotensive activity; or
a pharmaceutically acceptable salt thereof.

2. The peptide according to claim 1 or a pharmaceutically acceptable salt thereof, wherein n is 0.

3. The peptide according to claim 1 or 2 or a pharmaceutically acceptable salt thereof, wherein n and m are 0.

4. The peptide according to claim 1 or a pharmaceutically acceptable salt thereof, wherein the peptide described in (i) above consists of an amino acid sequence selected from the group consisting of SEQ ID NOS: 1, 8, 10 to 13, 18 to 23, and 25 to 43.

5. The peptide according to any one of claims 1 to 4 or a pharmaceutically acceptable salt thereof, wherein the hydrogen atom of the α-amino group of the amino acid at the amino terminus of $P1_n$ is substituted with an acetyl group or a pyroglutamyl group.

6. The peptide according to any one of claims 1 to 5 or a pharmaceutically acceptable salt thereof, wherein Y represents $NH_2$.

7. A pharmaceutical composition for the treatment of hypertension or disease caused by hypertension, which comprises a peptide according to any one of claims 1 to 6 or a pharmaceutically acceptable salt thereof.

8. A method for treating hypertension or disease caused by hypertension, which comprises administering the pharmaceutical composition according to claim 7 to an individual.

9. An antibody against the peptide according to any one of claims 1 to 6.

10. A method for determining the peptide according to any one of claims 1 to 6, which comprises detecting the peptide in a test sample using an antibody against the peptide.

11. A method for producing the peptide according to any one of claims 1 to 6 by genetic recombination technology, which comprises:

transforming a host cell with a vector containing DNA encoding the peptide;
culturing the obtained transformed cell and collecting a peptide of interest from the culture; and
carrying out a modification reaction, as necessary.

*Figure 1*

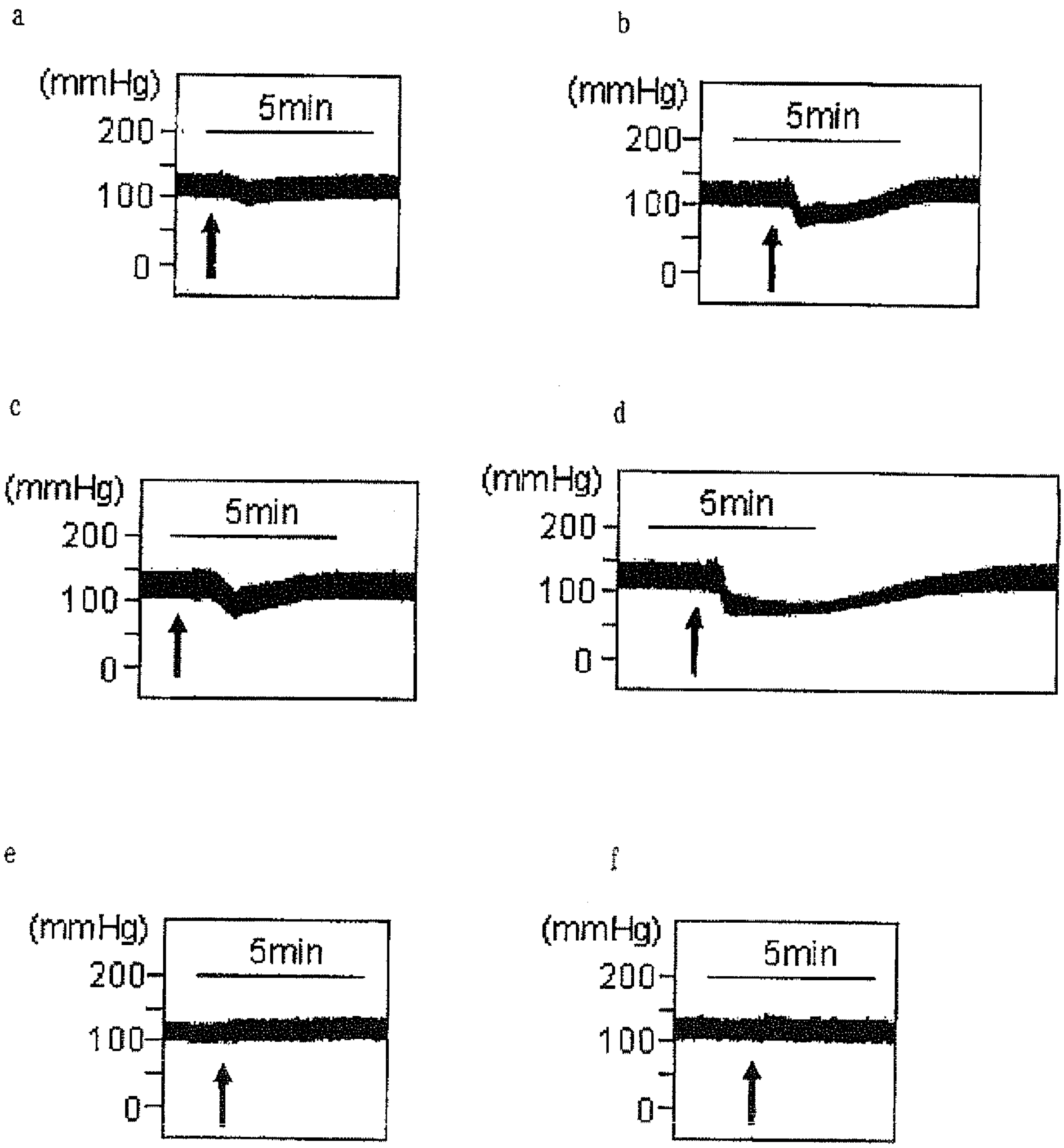
a
(mmHg)
200
100
0
5min
b
(mmHg)
200
100
0
5min
c
(mmHg)
200
100
0
5min
d
(mmHg)
200
100
0
5min
e
(mmHg)
200
100
0
5min
f
(mmHg)
200
100
0
5min

Figure 2

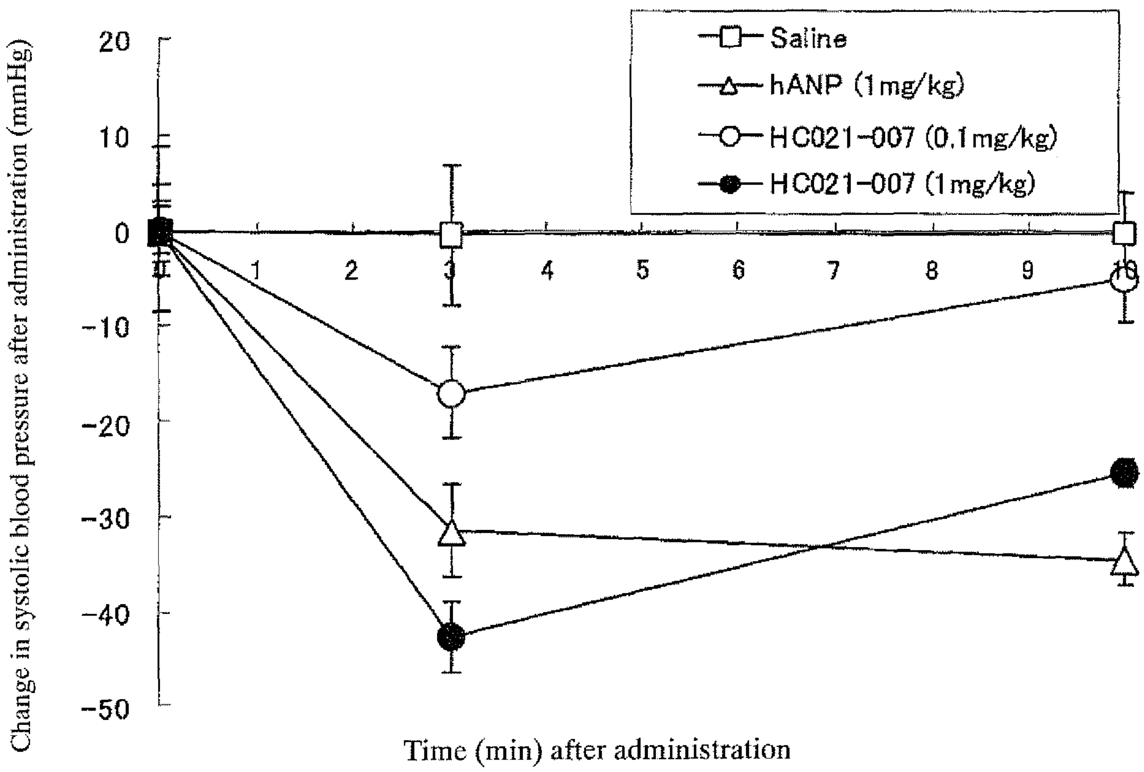
Saline
hANP (1mg/kg)
HC021-007 (0.1mg/kg)
HC021-007 (1mg/kg)
Change in systolic blood pressure after administration (mmHg)
20
10
0
-10
-20
-30
-40
-50
0
1
2
3
4
5
6
7
8
9
10
Time (min) after administration

*Figure 3*

a

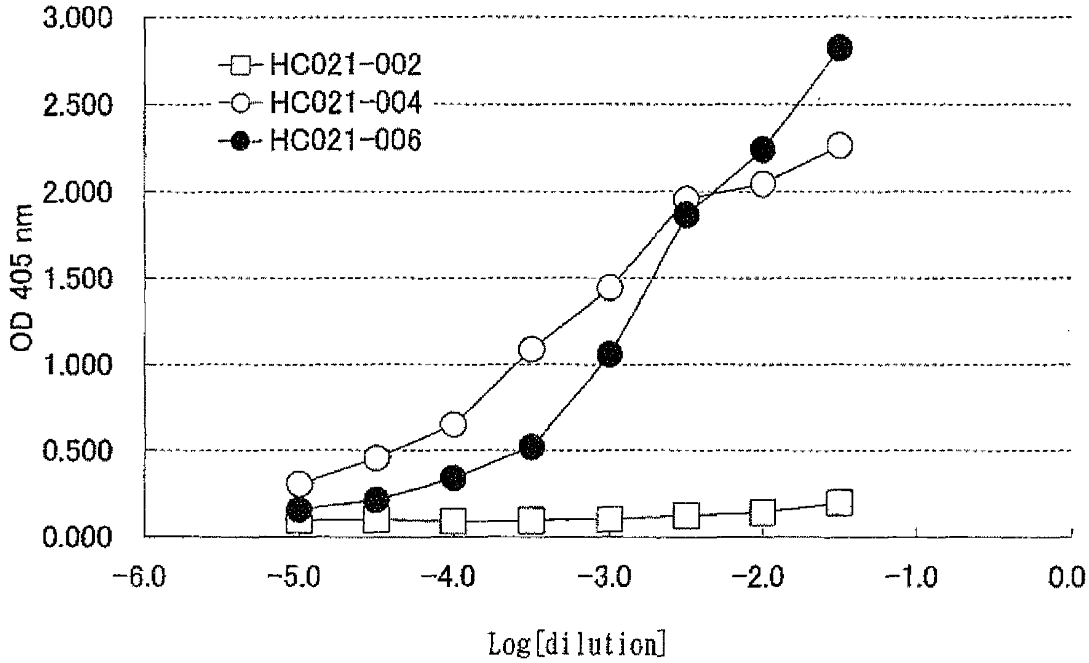


b

HC021-002 (SEQ ID NO: 7) LQAYFKGRGLDLGTFPNPFPTNENP

HC021-004 (SEQ ID NO: 8) NRDLFFRRLQAYFK-amide

HC021-006 (SEQ ID NO: 9) LQAYFK-amide

**INTERNATIONAL SEARCH REPORT**

International application No.
PCT/JP2008/061732

A. CLASSIFICATION OF SUBJECT MATTER
*C07K14/47*(2006.01)i, *A61K38/00*(2006.01)i, *A61P9/12*(2006.01)i, *A61P43/00* (2006.01)i, *C07K7/06*(2006.01)n, *C07K7/08*(2006.01)n

According to International Patent Classification (IPC) or to both national classification and IPC

B. FIELDS SEARCHED

Minimum documentation searched (classification system followed by classification symbols)
C07K14/47, A61K38/00, A61P9/12, A61P43/00, C07K7/06, C07K7/08

Documentation searched other than minimum documentation to the extent that such documents are included in the fields searched

Electronic data base consulted during the international search (name of data base and, where practicable, search terms used)
MEDLINE/CAplus/BIOSIS/WPIDS(STN), REGISTRY(STN), JSTPlus/JMEDPlus/ JST7580(JDreamII), GenBank/EMBL/DDBJ/GeneSeq, UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X<br>A | US 2005/0208602 A1 (Human Genome Sciences Inc.), 22 September, 2005 (22.09.05), (Family: none) | 9,10<br>1-7,11 |
| X<br>A | WO 2003/052377 A2 (Human Genome Sciences Inc.), 26 June, 2003 (26.06.03), & EP 1583947 A2 | 9,10<br>1-7,11 |
| X<br>A | WO 2002/002621 A2 (Zymogenetics, Inc.), 10 January, 2002 (10.01.02), & US 2002/110855 A1 & US 2005/214791 A1 | 9,10<br>1-7,11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 July, 2008 (14.07.08) | 22 July, 2008 (22.07.08) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2008/061732

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 8
because they relate to subject matter not required to be searched by this Authority, namely:
The invention of the above claim pertains to a method for treatment of a human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2001072800 A
- WO 2002002621 A
- WO 2002068579 A
- US 20050208602 A
- WO 2003052377 A


### Non-patent literature cited in the description


- **Kastin.** Handbook of biologically active peptide. Academic Press, 2006
- **Hosoda et al.** *J. Pharmacol. Sci.,* 2006, vol. 100, 398-410
- **Hinson et al.** *Endocrine Reviews,* 2000, vol. 21, 138-167
- **Morton et al.** *Nature,* 2006, vol. 4433, 289-295
- **Kangawa et al.** *Biochem. Biophys. Res. Commun.,* 1984, vol. 118, 131-139
- **Minamino et al.** *Biochem. Biophys. Res. Commun.,* 1985, vol. 130, 1078-1085
- **Miyata et al.** *Biochem. Biophys. Res. Commun.,* 1989, vol. 164, 567-574
- **Meunier et al.** *Nature,* 1995, vol. 377, 532-535
- **Kojima et al.** *Nature,* 1999, vol. 402, 656-660
- **Hinuma et al.** *Nature,* 1998, vol. 393, 272-276
- **Tatemoto et al.** *Biochem. Biophys. Res. Commun.,* 1998, vol. 251, 471-476
- **Johnson et al.** *J. Biol. Chem.,* 2003, vol. 278, 52172-52178
- **Verdonk et al.** *Assay Drug Dev. Technol.,* 2006, vol. 4, 609-619
- **Vassilatis et al.** *Proc. Natl. Acad. Sci.,* 2003, vol. 100, 4903-4908
- **Roh et al.** *J. Biol. Chem.,* 2004, vol. 279, 7264-7274
- **Hinuma et al.** *Nature Cell Biol.,* 2000, vol. 400, 703-708
- **Chartel et al.** *Proc. Natl. Acad. Sci.,* 2003, vol. 100, 15247-15252
- **Jiang et al.** *J. Biol. Chem.,* 2003, vol. 278, 27652-27657
- **Shichiri et al.** *Nat. Med.,* 2003, vol. 9, 1166-1172
- **Mirabeau et al.** *Genome Research,* 2007, vol. 17, 320-327
- **Seidah et al.** *Brain Res.,* 1999, vol. 848, 45-62
- **Steiner.** *Curr. Opin. Chem. Biol.,* 1998, vol. 2, 31-39
- **Duckert et al.** *Protein Eng. Des. Sel.,* 2004, vol. 17, 107-112
- *Nature Biotechnology,* 2001, vol. 19 (5), 440-445
- *Genome Research,* 2003, vol. 13 (10), 2265-2270
- Seikagaku Jikken Koza 1. Tanpakushitsu no Kagaku. Tokyo Kagaku Dojin, vol. 4
- Zoku Iyakuhin no Kaihatsu 14. Pepuchido Gosei. Hirokawa Shoten